(19) Europäisches Patentamt
European Patent Office
Office européen des brevets

(11) **EP 1 399 568 B1**

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **02716713.9**

(22) Anmeldetag: **01.02.2002**

(51) Int Cl.[7]: **C12N 15/57**, C12N 9/76, C12N 1/19

(86) Internationale Anmeldenummer:
**PCT/EP2002/001072**

(87) Internationale Veröffentlichungsnummer:
**WO 2002/061064 (08.08.2002 Gazette 2002/32)**

(54) **VERFAHREN ZUR HERSTELLUNG VON REKOMBINANTEM TRYPSIN**

METHOD FOR PRODUCING RECOMBINANT TRYPSIN

PROCEDE DE PREPARATION DE TRYPSINE RECOMBINEE

(84) Benannte Vertragsstaaten:
**AT BE CH CY DE DK ES FI FR GB GR IE IT LI LU MC NL PT SE TR**

(30) Priorität: **01.02.2001 EP 01102342**

(43) Veröffentlichungstag der Anmeldung:
**24.03.2004 Patentblatt 2004/13**

(73) Patentinhaber:
- **Roche Diagnostics GmbH**
  **68305 Mannheim (DE)**
  Benannte Vertragsstaaten:
  **DE**
- **F. HOFFMANN-LA ROCHE AG**
  **4070 Basel (CH)**
  Benannte Vertragsstaaten:
  **AT BE CH CY DK ES FI FR GB GR IE IT LI LU MC NL PT SE TR**

(72) Erfinder:
- **MÜLLER, Rainer**
  **82377 Penzberg (DE)**
- **GLASER, Stephan**
  **82402 Seeshaupt (DE)**
- **GEIPEL, Frank**
  **82377 Penzberg (DE)**
- **THALHOFER, Johann-Peter**
  **82362 Weilheim (DE)**
- **REXER, Bernhard**
  **82362 Weilheim (DE)**
- **SCHNEIDER, Claus**
  **69214 Eppelheim (DE)**
- **RATKA, Michael**
  **68161 Mannheim (DE)**
- **RONNING, Stephanie**
  **82377 Penzberg (DE)**
- **ECKSTEIN, Hellmut**
  **82362 Weilheim (DE)**
- **GIESSEL, Claudia**
  **83677 Greiling (DE)**

(74) Vertreter: **Weiss, Wolfgang et al**
**Weickmann & Weickmann**
**Patentanwälte**
**Postfach 86 08 20**
**81635 München (DE)**

(56) Entgegenhaltungen:
**EP-A- 0 597 681**
**EP-A- 0 699 687**
**WO-A-00/17332**
**WO-A-01/55429**
**WO-A-97/00316**

- **J SAMBROOK AND D RUSSELL: "Molecular Cloning" 2001 , COLD SPRING HARBOR LABORATORY PRESS , NEW YORK XP002229999 Seite 7.2A -Seite 7.3A**
- **DATABASE SWISSPROT [Online] 21. Juli 1986 (1986-07-21) "Trypsin precursor" Database accession no. P00761 XP002230000**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 1 399 568 B1

SEQUENCE LISTING

<110> Roche Diagnostics GmbH

<120> Verfahren zur Herstellung von rekombinanem Trypsin

<130> 5515/00/EP

<140>
<141>

<150> 01102342.1
<151> 2001-02-01

<160> 23

<170> PatentIn Ver. 2.1

<210> 1
<211> 247
<212> PRT
<213> Porc

<400> 1

```
Ile Pro Asn Thr Phe Val Leu Leu Ala Leu Leu Gly Ala Ala Val Ala
 1               5                  10                  15

Phe Pro Thr Asp Asp Asp Asp Lys Ile Val Gly Gly Tyr Thr Cys Ala
            20                  25                  30

Ala Asn Ser Ile Pro Tyr Gln Val Ser Leu Asn Ser Gly Ser His Phe
        35                  40                  45

Cys Gly Gly Ser Leu Ile Asn Ser Gln Trp Val Val Ser Ala Ala His
    50                  55                  60

Cys Tyr Lys Ser Arg Ile Gln Val Arg Leu Gly Glu His Asn Ile Asp
65                  70                  75                  80

Val Leu Glu Gly Asn Glu Gln Phe Ile Asn Ala Ala Lys Ile Ile Thr
                85                  90                  95

His Pro Asn Phe Asn Gly Asn Thr Leu Asp Asn Asp Ile Met Leu Ile
            100                 105                 110

Lys Leu Ser Ser Pro Ala Thr Leu Asn Ser Arg Val Ala Thr Val Ser
        115                 120                 125

Leu Pro Arg Ser Cys Ala Ala Ala Gly Thr Glu Cys Leu Ile Ser Gly
    130                 135                 140

Trp Gly Asn Thr Lys Ser Ser Gly Ser Ser Tyr Pro Ser Leu Leu Gln
145                 150                 155                 160

Cys Leu Lys Ala Pro Val Leu Ser Asp Ser Ser Cys Lys Ser Ser Tyr
                165                 170                 175

Pro Gly Gln Ile Thr Gly Asn Met Ile Cys Val Gly Phe Leu Glu Gly
            180                 185                 190

Gly Lys Asp Ser Cys Gln Gly Asp Ser Gly Gly Pro Val Val Cys Asn



        195                 200                 205

Gly Gln Leu Gln Gly Ile Val Ser Trp Gly Tyr Gly Cys Ala Gln Lys
    210                 215                 220

Asn Lys Pro Gly Val Tyr Thr Lys Val Cys Asn Tyr Val Asn Trp Ile
225                 230                 235                 240

Gln Gln Thr Ile Ala Ala Asn
                245
```

<210> 2
<211> 744
<212> DNA
<213> Artificial Sequence

<220>
<223> Description of Artificial Sequence: Synthetic Gene

<400> 2

```
attccaaata cttttgtttt gttggctttg ttgggtgctg ctgttgcttt tccaactgat 60
gatgatgata aaattgttgg tggttatact tgtgctgcta attctattcc atatcaagtt 120
tctttaaatt ctggttctca tttttgtggt ggttctttga ttaattctca atgggttgtt 180
tctgctgctc attgttacaa atcaagaatc caagttagat tgggtgaaca taatattgat 240
gttttggaag gtaatgaaca atttattaat gctgctaaaa ttattactca tccaaatttt 300
aatggtaata ctttggataa tgatattatg ttgattaaat tgtcttctcc agctacttta 360
aattcaagag ttgctactgt ttctttgcca agatcttgtg ctgctgctgg tactgaatgt 420
ttgatttctg gttggggtaa tactaaatct tctggttctt cttatccatc tttgttgcaa 480
tgtttgaaag ctccagtttt gtctgattct tcttgtaaat cttcttaccc aggtcaaatt 540
actggtaata tgatttgtgt tggtttttttg gaaggtggta aagattcttg tcaaggtgat 600
tctggtggtc cagttgtttg taatggtcaa ttgcaaggta ttgtttcttg gggttatggt 660
tgtgctcaaa aaaataaacc aggtgtttac actaaagttt gtaattatgt taattggatt 720
caacaaacta ttgctgctaa ttag                                        744
```

<210> 3
<211> 56
<212> DNA
<213> Artificial Sequence

<220>
<223> Description of Artificial Sequence: Primer

<400> 3

```
gcggaattca ttccaaatac ttttgttttg ttggctttgt tgggtgctgc tgttgc     56
```

<210> 4
<211> 54
<212> DNA
<213> Artificial Sequence

<220>
<223> Description of Artificial Sequence: Primer

<400> 4

```
ccaccaacaa ttttatcatc atcatcagtt ggaaaagcaa cagcagcacc caac       54
```

<210> 5
<211> 62
<212> DNA
<213> Artificial Sequence

<220>
<223> Description of Artificial Sequence: Primer

<400> 5

```
gatgataaaa ttgttggtgg ttatacttgt gctgctaatt ctattccata tcaagtttct 60
tt                                                                62
```

<210> 6
<211> 64

<212> DNA
<213> Artificial Sequence

<220>
<223> Description of Artificial Sequence: Primer

<400> 6

```
gaattaatca aagaaccacc acaaaaatga gaaccagaat ttaaagaaac ttgatatgga 60
atag                                                              64
```

<210> 7
<211> 64
<212> DNA
<213> Artificial Sequence

<220>
<223> Description of Artificial Sequence: Primer

<400> 7

```
ggtggttctt tgattaattc tcaatgggtt gtttctgctg ctcattgtta caaatcaaga 60
atcc                                                              64
```

<210> 8
<211> 62
<212> DNA
<213> Artificial Sequence

<220>
<223> Description of Artificial Sequence: Primer

<400> 8

```
ccttccaaaa catcaatatt atgttcaccc aatctaactt ggattcttga tttgtaacaa 60
tg                                                                62
```

<210> 9
<211> 63
<212> DNA
<213> Artificial Sequence

<220>
<223> Description of Artificial Sequence: Primer

<400> 9

```
taatattgat gttttggaag gtaatgaaca atttattaat gctgctaaaa ttattactca 60
tcc                                                               63
```

<210> 10
<211> 62
<212> DNA
<213> Artificial Sequence

<220>
<223> Description of Artificial Sequence: Primer

<400> 10

caacataata tcattatcca aagtattacc attaaaattt ggatgagtaa taattttagc 60
ga 62

<210> 11
<211> 64
<212> DNA
<213> Artificial Sequence

<220>
<223> Description of Artificial Sequence: Primer

<400> 11

ctttggataa tgatattatg ttgattaaat tgtcttctcc agctacttta aattcaagag 60
ttgc 64

<210> 12
<211> 58
<212> DNA
<213> Artificial Sequence

<220>
<223> Description of Artificial Sequence: Primer

<400> 12

ccagcagcag cacaagatct tggcaaagaa acagtagcaa ctcttgaatt taaagtag 58

<210> 13
<211> 61
<212> DNA
<213> Artificial Sequence

<220>
<223> Description of Artificial Sequence: Primer

<400> 13

cttgtgctgc tgctggtact gaatgtttga tttctggttg gggtaatact aaatcttctg 60
g 61

<210> 14
<211> 58
<212> DNA
<213> Artificial Sequence

<220>
<223> Description of Artificial Sequence: Primer

<400> 14

gagctttcaa acattgcaac aaagatggat aagaagaacc agaagattta gtattacc 58

<210> 15
<211> 60
<212> DNA
<213> Artificial Sequence

<220>
<223> Description of Artificial Sequence: Primer

<400> 15

gttgcaatgt ttgaaagctc cagttttgtc tgattcttct tgtaaatctt cttacccagg 60

<210> 16
<211> 59
<212> DNA
<213> Artificial Sequence

<220>
<223> Description of Artificial Sequence: Primer

<400> 16

ccaaaaaacc aacacaaatc atattaccag taatttgacc tgggtaagaa gatttacaa 59

<210> 17
<211> 60
<212> DNA
<213> Artificial Sequence

<220>
<223> Description of Artificial Sequence: Primer

<400> 17

gatttgtgtt ggtttttgg aaggtggtaa agattcttgt caaggtgatt ctggtggtcc 60

<210> 18
<211> 57
<212> DNA
<213> Artificial Sequence

<220>
<223> Description of Artificial Sequence: Primer

<400> 18

ccaagaaaca ataccttgca attgaccatt acaaacaact ggaccaccag aatcacc 57

<210> 19
<211> 51
<212> DNA
<213> Artificial Sequence

<220>
<223> Description of Artificial Sequence: Primer

<400> 19

gcaaggtatt gtttcttggg gttatggttg tgctcaaaaa aataaaccag g 51

<210> 20
<211> 90
<212> DNA
<213> Artificial Sequence

<220>
<223> Description of Artificial Sequence: Primer

<400> 20

gcgtctagac taattagcag caatagtttg ttgaatccaa ttaacataat tacaaacttt 60
agtgtaaaca cctggtttat ttttttgagc 90

<210> 21
<211> 48
<212> DNA
<213> Artificial Sequence

<220>
<223> Description of Artificial Sequence: Primer

<400> 21

ccgggaattc gatgatgatg ataaaattgt tggtggttat acttgtgc 48

<210> 22
<211> 687
<212> DNA
<213> Artificial Sequence

<220>
<223> Description of Artificial Sequence: Synthetic Gene

<400> 22

```
gatgatgatg ataaaattgt tggtggttat acttgtgctg ctaattctat tccatatcaa 60
gtttctttaa attctggttc tcatttttgt ggtggttctt tgattaattc tcaatgggtt 120
gtttctgctg ctcattgtta caaatcaaga atccaagtta gattgggtga acataatatt 180
gatgttttgg aaggtaatga acaatttatt aatgctgcta aaattattac tcatccaaat 240
tttaatggta atactttgga taatgatatt atgttgatta aattgtcttc tccagctact 300
ttaaattcaa gagttgctac tgtttctttg ccaagatctt gtgctgctgc tggtactgaa 360
tgtttgattt ctggttgggg taatactaaa tcttctggtt cttcttatcc atctttgttg 420
caatgtttga aagctccagt tttgtctgat tcttcttgta aatcttctta cccaggtcaa 480
attactggta atatgatttg tgttggtttt ttggaaggtg gtaaagattc ttgtcaaggt 540
gattctggtg gtccagttgt ttgtaatggt caattgcaag gtattgtttc ttggggttat 600
ggttgtgctc aaaaaaataa accaggtgtt tacactaaag tttgtaatta tgttaattgg 660
attcaacaaa ctattgctgc taattag                                    687
```

<210> 23
<211> 228
<212> PRT
<213> Artificial Sequence

<220>
<223> Description of Artificial Sequence: Shortened Trypsinogen

<400> 23

```
Asp Asp Asp Asp Lys Ile Val Gly Gly Tyr Thr Cys Ala Ala Asn Ser
1               5                   10                  15

Ile Pro Tyr Gln Val Ser Leu Asn Ser Gly Ser His Phe Cys Gly Gly
            20                  25                  30

Ser Leu Ile Asn Ser Gln Trp Val Val Ser Ala Ala His Cys Tyr Lys
        35                  40                  45

Ser Arg Ile Gln Val Arg Leu Gly Glu His Asn Ile Asp Val Leu Glu
    50                  55                  60

Gly Asn Glu Gln Phe Ile Asn Ala Ala Lys Ile Ile Thr His Pro Asn
65                  70                  75                  80

Phe Asn Gly Asn Thr Leu Asp Asn Asp Ile Met Leu Ile Lys Leu Ser
                85                  90                  95

Ser Pro Ala Thr Leu Asn Ser Arg Val Ala Thr Val Ser Leu Pro Arg
```

```
                100                 105                 110
Ser Cys Ala Ala Ala Gly Thr Glu Cys Leu Ile Ser Gly Trp Gly Asn
        115                 120                 125

Thr Lys Ser Ser Gly Ser Ser Tyr Pro Ser Leu Leu Gln Cys Leu Lys
    130                 135                 140

Ala Pro Val Leu Ser Asp Ser Ser Cys Lys Ser Ser Tyr Pro Gly Gln
145                 150                 155                 160

Ile Thr Gly Asn Met Ile Cys Val Gly Phe Leu Glu Gly Gly Lys Asp
                165                 170                 175

Ser Cys Gln Gly Asp Ser Gly Gly Pro Val Val Cys Asn Gly Gln Leu
            180                 185                 190

Gln Gly Ile Val Ser Trp Gly Tyr Gly Cys Ala Gln Lys Asn Lys Pro
        195                 200                 205

Gly Val Tyr Thr Lys Val Cys Asn Tyr Val Asn Trp Ile Gln Gln Thr
    210                 215                 220

Ile Ala Ala Asn
225
```

## Beschreibung

Die Erfindung betrifft ein Verfahren zur rekombinanten Herstellung von Trypsin. Vorzugsweise wird hierzu ein in der Propeptidsequenz verkürztes Trypsinogen in einer rekombinanten Wirtszelle exprimiert und in ungespaltener Form ins Kulturmedium sekretiert. Anschließend erfolgt eine kontrollierte Abspaltung der Propeptidsequenz unter Bildung von aktivem Trypsin.

Trypsin ist eine Serinprotease, die eine hydrolytische Spaltung von Peptiden an der Carboxylgruppe der basischen Aminosäuren Arginin und Lysin katalysiert (Keil B., 1971). Trypsin aus Rinderpankreas war eines der ersten proteolytischen Enzyme, die in reiner Form und ausreichenden Mengen für exakte chemische und enzymatische Studien verwendet werden konnten (Northrop et al., 1948). Später konnten Proteasen, die der Trypsinfamilie zugeordnet werden können, auch aus anderen höheren Vertebraten (Schwein - Charles et al. 1963 / Schaf - Bricteux-Gregoire et al.(1966); Travis (1968) / Truthahn - Ryan (1965) / Mensch - Travis et al. (1969)und andere) isoliert werden. In dieser Zeit wurden auch die ersten Enzyme, die der Trypsinfamilie angehören, aus zwei Arten von Streptomyces (Morihara and Tsuzuki (1968); Trop and Birk (1968); Wählby and Engström (1968); Wählby (1968; Jurasek et al. (1969)) isoliert.

In den Pankreaszellen von Vertebraten wird das Enzym als inaktiver Precursor, Trypsinogen, synthetisiert und anschliessend durch Abspaltung des Propeptids in die aktive Form überführt (Northrop et al. (1948), Desnuelle (1959)). Die Aktivierung der ersten Trypsinogenmoleküle erfolgt naturgemäß durch die Enteropeptidase Enterokinase, die die Peptidbindung zwischen ($Asp_4$)-Lys-↓-Ile hydrolysiert, wodurch eine Abspaltung des Propeptids erfolgt (Keil (1971)). Die Erkennungssequenz der Enterokinase ($Asp_4$)-Lys findet man entsprechend in fast allen bislang bekannten Trypsinogenmolekülen direkt am C-Terminus des Propeptids (Light et al. (1980)). Der Aktivierungsprozess kann bei physiologischen pH-Werten jedoch auch autokatalytisch verlaufen, da sich C-terminal der Enterokinase-Erkennungssequenz ein Lysin befindet und somit die Hydrolyse der Lys-↓-Ile-Peptidbindung auch durch Trypsin erfolgen kann (Light et al. (1980)).

Wegen der guten Verfügbarkeit aus verschiedenen Säugern, der hohen Spezifität (spaltet nur C-terminal von Lysin oder Arginin) bei gleichzeitig hoher spezifischen Aktivität (~150 U/mg) und seiner hohen Lagerstabilität ist Trypsin seit je her eine interessante Protease für biotechnologische Anwendungen. Zum Einsatz kommt Trypsin hauptsächlich bei tryptischer Spaltung von Peptiden in kleine Teilstücke zur Sequenzierung, zur Ablösung adherenter Zellen von beschichteten Zellkulturschalen und zur Spaltung von Fusionsproteinen in das Zielpeptid und den Fusionsanteil, zur Aktivierung von Propeptiden (z.B. Trypsinogen zu Trypsin) und zur rekombinanten Herstellung von Peptidhormonen (z.B. Proinsulin zu Insulin, vgl. hierzu WO 99/10503). Außerdem ist Trypsin Bestandteil einiger Arzneimittel (Salben, Dragees und zu inhalierenden Aerosolen (Rote Liste, 1997; The United States Pharmacopeia, The National Formulary, USP23-NF18, 1995)). Da in vielen Fällen der Einsatz von Enzymen aus tierischen Quellen nicht mehr zulässig ist (mögliche Kontamination mit infektiösen Agenzien), müssen rekombinante Trypsinmoleküle aus mikrobiellen Wirten für die gewünschten biotechnologischen Anwendungen zur Verfügung gestellt werden.

Es sind mehrere Verfahren zur rekombinanten Herstellung von Trypsin aus unterschiedlichen Organismen bekannt.

Graf, L. et al (1987 und 1988) beschreiben die Expression und Sekretion von Ratten-Trypsin und Trypsinogenmutanten in E.coli. Zur Sekretion der Trypsinogenmoleküle in das Periplasma von E.coli wird die native Trypsinogen-Signalsequenz durch die Signalsequenz der bakteriellen alkalischen Phosphatase (phoA) ausgetauscht. Die sekretierten inaktiven Trypsinogenmoleküle werden aus dem Periplasma isoliert und durch enzymatische Spaltung mittels gereinigter Enterokinase aktiviert.

Von Vasquez, J.R. et al. (1989) wird die Expression und Sekretion von anionischem Ratten-Trypsin und Trypsinmutanten in E.coli beschrieben. Zur Expression und Sekretion der aktiven Trypsinmoleküle in das Periplasma von E.coli wird das native Trypsinogen Prepro-Segment (Signalsequenz und Aktivierungspeptid) durch die Signalsequenz der bakteriellen alkalischen Phosphatase (phoA) ersetzt und der durch Phosphat regulierbare phoA-Promotor verwendet. Aktives Trypsin wird aus dem Periplasma isoliert. Die Ausbeute ist jedoch sehr gering (ca. 1 mg/l).

Higaki, J.N. et al. (1989) beschreiben die Expression und Sekretion von Trypsin und Trypsinmutanten in das Periplasma von E.coli unter Verwendung des tac- Promotors und der S.typhimurium hisJ Signalsequenz. Die Ausbeute an aktivem Trypsin beträgt ca. 0,3 mg/l. Durch Hochzelldichtefermentation kann die Volumenausbeute von aktivem anionischem Rattentrypsin auf ca. 50 mg/l gesteigert werden (Yee, L. und Blanch, H.W., (1993)). Die Autoren weisen jedoch auf Probleme bei der Expression und Sekretion von aktivem Trypsin in E.coli hin. Enzymatisch aktives Trypsin entsteht im Periplasma von E.coli nach Abspaltung der Signalsequenz und nativer Trypsinproteinfaltung unter Ausbildung von 6 Disulfidbrücken. Die Bildung von aktivem Trypsin ist für die Zelle toxisch, da aktives Trypsin die periplasmatischen E.coli Proteine hydrolysiert, wodurch die Zellen lysieren. Zudem scheint die Proteinfaltung von Trypsin, im speziellen die korrekte native Ausbildung der 6 Disulfidbrücken im Periplasma von E.coli erschwert zu sein. Das System ist nicht geeignet zur Isolierung grösserer Mengen an Trypsin (> 10 mg; Willett, W.S. et al., (1995)).

Zur Herstellung größerer Mengen Trypsin (50-100 mg) für röntgenkristallographische Untersuchungen wird eine inaktive Trypsinogen-Vorstufe in Hefe unter Kontrolle eines regulierbaren ADH/GAPDH-Promotors und durch Fusion mit der Hefe α-Faktor Leadersequenz sekretiert. Das in das Medium sekretierte Expressionsprodukt wird in vitro mittels Enterokinase quantitativ in Trypsin überführt. Die Ausbeute beträgt 10-15 mg/l (Hedstrom, L. et al. (1992)).

In EP 0 597 681 werden DNA-Sequenzen beschrieben, die für reifes Rinder-Trypsin bzw. Rindertrypsinogen mit vorangehendem Methioninrest kodieren. Zudem wird die Expression in E.coli beschrieben, jedoch nicht die Strategie erläutert, wie aktives Trypsin in E.coli entsteht.

In WO 97/O0316 wird ein Verfahren zur Herstellung von Trypsin aus Schweinepankreas oder eines Derivats durch ein rekombinantes Verfahren in Aspergillus beschrieben. Zur Transformation wird ein Vektor verwendet, welcher für Trypsinogen oder ein Derivat davon kodiert, welches N-terminal an ein funktionsfähiges Signalpeptid fusioniert ist. Hefekulturen erreichen jedoch im Vergleich zu Aspergüluskulturen höhere Biomasse-Konzentrationen und wachsen deutlich schneller, so dass die spezifische Expressionsleistung pro Hefezelle geringer sein kann als bei Aspergilluszellen, um die Ausbeuten für ein wirtschaftliches Expressionsverfahren zu erreichen.

In WO 99/10503 ist ein Verfahren zur rekombinanten Herstellung einer zymogenen Vorstufe von Proteasen beschrieben, die eine natürlicherweise nicht vorkommende autokatalytische Spaltstelle enthält. Das Verfahren umfasst eine Expression der zymogenen Vorstufe in E.coli in Form von Inclusion Bodies mit anschliessender Reinigung und Naturierung der unter Bedingungen, bei denen der Proteaseteil der zymogenen Vorstufe in seiner natürlichen Konformation entsteht und die Spaltung der naturierten, zymogenen Vorstufe dann autokatalytisch erfolgt. Nachteil dieses Verfahrens sind die oftmals hohen Verluste bei der Naturierung und die großen Volumina bei großtechnischer Herstellung.

In WO 00/17332 ist die rekombinante Herstellung von Trypsinanaloga in Pichia pastoris beschreiben. Zur Transformation wird ein Vektor verwendet, welcher für ein Trypsinogenanalogon (Derivat von Rindertrypsinogen) kodiert, in welchen durch Mutation die Aminosäure Lysin am C-Terminus des Propeptids gegen eine andere Aminosäure (außer Arginin oder Lysin) ausgetauscht wurde und die N-terminal an ein funktionsfähiges Signalpeptid fusioniert sind. Dabei werden die Trypsinanaloga löslich in das Medium sekretiert und durch die eingebaute Mutation auch bei höheren pH-Werten des Fermentationsprozesses nicht durch unerwünschte Autokatalyse aktiviert und weiter abgebaut. Zur Aktivierung wird dann eine Aminopeptidase verwendet. Nachteil dieses Verfahrens ist jedoch die Abreicherung der zusätzlichen Aminopeptidase, die für die weitere Verwendung des Trypsins im Zielprozess nachteilige Nebenaktivitäten haben kann.

In WO01/55429 wird die Expression von natürlich vorkommenden Trypsinogen-Genen in Pichia pastoris beschrieben. Zur Vermeidung der autokatalytischen Aktivierung wird die Fermentation im Beispiel in dem wenig sauren pH-Bereich bei ca. 6 durchgeführt. Damit liegt man außerhalb des optimalen pH-Bereichs, der eine autokatalytische Aktivierung während langer Laufzeiten in der Expressionsphase verhindert. Nachteil des Verfahrens sind außerdem die nicht für die Expression in Pichia pastoris codonoptimierten Trypsinogen-Gene und die damit verbundenen geringeren Expressionsausbeuten.

Aufgabe der Erfindung ist die Bereitstellung eines Verfahrens zur rekombinanten Herstellung von Trypsin, bei dem die Nachteile des Standes der Technik mindestens teilweise beseitigt sind und das auf einfache Weise die Gewinnung von aktivem Trypsin in hoher Ausbeute und Aktivität erlaubt. Insbesondere soll als Zwischenprodukt Trypsinogen in löslicher Form aus dem Kulturmedium des Expressionswirts isolierbar sein und während der Fermentation und Aufreinigung weitgehend keiner autokatalytischen Aktivierung unterliegen. Außerdem soll die anschließende Aktivierung des Trypsinogens autokatalytisch und/oder durch Zugabe von rekombinantem Trypsin möglich sein.

Die der Erfindung zugrunde liegende Aufgabe wird gelöst durch ein Verfahren zur rekombinanten Herstellung von Trypsin, umfassend die Schritte:

a) Transformieren einer Wirtszelle mit einer rekombinanten Nukleinsäure, die für ein Trypsinogen mit einer Enterokinase-Erkennungsstelle in der Propeptidsequenz in sekretierbarer Form, vorzugsweise fusioniert mit einem die Sekretion vermittelnden Signalpeptid codiert, unter der Veraussetzung, daß die rekombinante Nukleinsaure nicht mit einem naturlich vorkommenden TrypsinogenGen identisch ist,
b) Kultivieren der Wirtszelle unter Bedingungen, bei denen eine Expression der rekombinanten Nukleinsäure und eine Sekretion des Expressionsprodukts in das Kulturmedium erfolgt, wobei die Bedingungen so gewählt werden, dass eine autokatalytische Abspaltung der Propeptidsequenz zumindest weitgehend verhindert wird,
c) Isolieren des Expressionsprodukts aus dem Kulturmedium,
d) Abspalten der Propeptidsequenz, wobei aktives Trypsin gebildet wird und
e) gegebenenfalls Abtrennen nicht gespaltener Trypsinogenmoleküle von aktivem Trypsin.

Die im erfindungsgemäßen Verfahren verwendete Wirtszelle kann eine eukaryontische oder prokaryontische Wirtszelle sein, wie aus dem Stand der Technik bekannt. Vorzugweise ist die Wirtszelle eine eukaryontische Zelle, besonders bevorzugt eine Pilzzelle und am meisten bevorzugt eine Hefezelle. Geeignete Beispiele für Hefezellen sind Pichia pastoris, Hansenula polymorpha, Saccharomyces cerevisiae, Schizosaccharomyces pombae, wobei bevorzugt methylotrophe Hefen wie Pichia pastoris oder Hansenula polymorpha, insbesondere Pichia pastoris verwendet werden.

Zweckmäßigerweise wird eine solche Wirtszelle gewählt, die zur Expression der rekombinanten Nukleinsäure und zur Sekretion des Expressionsprodukts in das Kulturmedium in der Lage ist unter Bedingungen, bei denen eine autokatalytische Abspaltung der Propeptidsequenz zumindest weitgehend, vorzugsweise im wesentlichen quantitativ verhindert wird. Solche Bedingungen umfassen günstigerweise einen sauren pH-Wert des Kulturmediums, besonders bevorzugt einen pH-Wert im Bereich zwischen 3 und 4. Trypsinogen ist bei sauren pH-Werten, insbesondere bei pH-Werten bis zu 4 stabil, während im neutralen oder alkalischen Medium eine autokatalytische Aktivierung zu Trypsin stattfindet (Keil, B. (1971)).

Durch die beim erfindungsgemäßen Verfahren erfolgende Kultivierung unter geeigneten Bedingungen wird eine vorzeitige Aktivierung des von der Wirtszelle sekretierten rekombinanten Trypsinogens verhindert, wodurch auch der weitere Abbau des resultierenden Trypsins bis hin zu inaktiven Peptiden weitgehend verhindert werden kann. Die Stabilität des rekombinanten Trypsinogens unter den Kultivierungsbedingungen ist bedeutsam, da die Expression mit einer Sekretion einhergehen soll. Unter Sekretion aus der Wirtszelle ist dabei das Ausschleusen des Trypsinogens aus dem Zytoplasma durch die Zellmembran in das Kulturmedium zu verstehen. Dies geschieht üblicherweise durch N-terminale Fusion des Trypsinogens mit einem funktionsfähigen Signalpeptid. Beispiele für geeignete Signalpeptide sind bekannte Signalpeptide aus Hefe, besonders bevorzugt das Signalpeptid des $\alpha$-Faktors aus Saccharomyces cerevisiae. Selbstverständlich können jedoch auch andere Signalpeptide eingesetzt werden, z.B. das Signalpeptid, das natürlicherweise die Sekretion von Trypsinogen steuert.

Um die Expressionsausbeute des rekombinanten Trypsins zu verbessern, wird vorzugsweise eine rekombinante Nukleinsäure mit einer für die jeweilige Wirtszelle optimierten Codonnutzung verwendet. Für eine Hefezelle wird demnach günstigerweise eine Nukleinsäure eingesetzt, die eine für Hefe optimierte Codonnutzung aufweist. Die Herstellung solcher Nukleinsäuren mit optimierter Codonnutzung kann beispielsweise durch parallele Synthese einzelner Oligonukleotide und anschließende Zusammenlagerung erfolgen.

Das erfindungsgemäße Verfahren ist grundsätzlich zur Herstellung beliebiger Arten von rekombinantem Trypsin geeignet, sofern das jeweilige als Expressionsprodukt von der Wirtszelle sekretierte Trypsinogen unter den Kultivierungsbedingungen im wesentlichen stabil ist. Vorzugsweise wird das Verfahren zur Herstellung von Trypsin aus Vertebraten, insbesondere Säugern wie Schweinen, Schaf oder Mensch verwendet. Besonders bevorzugt wird Schweine-Trypsin hergestellt.

Weiterhin ist bevorzugt, dass man eine rekombinante Nukleinsäure verwendet, die für ein Trypsinogen mit verkürzter Propeptidsequenz codiert. Unter Trypsinogen ist ein Protein zu verstehen, das zwar weitgehend eine korrekte Proteinstruktur ausgebildet hat, aber im Gegensatz zu aktivem Trypsin keine oder nur sehr geringe proteolytische Aktivität hat, die günstigerweise mindestens um den Faktor 5 und besonders bevorzugt mindestens um den Faktor 10 geringer als die proteolytische Aktivität der aktiven Form ist. Der Propeptidanteil ist von der natürlichen Länge, z.B. 25 Aminosäuren bei Schweine-Trypsinogen verkürzt, vorzugsweise auf eine Propeptidsequenz die ausschließlich aus einer Erkennungssequenz für eine Enteropeptidase, z.B. Enterokinase besteht, z.B. eine Aminosäuresequenz Asp-Asp-Asp-Asp-Lys. Gegebenenfalls können N-terminal der Enterokinase-Erkennungssequenz einige weitere, durch die Klonierung bedingte Aminosäuren, z.B. bis zu 5 Aminosäuren angefügt werden.

Es wurde gefunden, dass die Expression eines erfindungsgemäß verkürzten rekombinanten Trypsinogens mit den klonierungsbedingt N-terminalen an die Enterokinase-Erkennungsstelle angefügten Aminosäuren Glu-Phe zu signifikant höheren Ausbeuten als die Expression des natürlichen Trypsinogens beim erfindungsgemäßen Verfahren erfolgt. In einer besonders bevorzugten Ausführungsform des erfindungsgemäßen Verfahrens wird daher eine rekombinante Nukleinsäure mit der in SEQ. ID NO. 22 gezeigten Nukleotidsequenz verwendet, die für eine Schweine-Trypsinogen mit verkürzter Propeptidsequenz codiert.

Die Expression des rekombinanten Trypsinogens beim erfindungsgemäßen Verfahren wird vorzugsweise durch eine induzierbare Expressionskontrollsequenz gesteuert. So kann das Wachstum der Wirtszelle vor Induktion der Expression unter Bedingungen erfolgen, die für das Wachstum der Wirtszelle günstig bzw. optimal sind. So kann beispielsweise das Wachstum bei pH 5-7, insbesondere pH 5-6, bis zu einer vorbestimmten optischen Dichte erfolgen, wobei die regulierbare Expressionskontrollsequenz reprimiert ist. Die Induzierung der Expression kann durch Temperaturänderung und/oder durch Zugabe eines Induktors erfolgen. Ein Beispiel für eine bevorzugte Expressionskontrollsequenz ist der durch Methanol induzierbare AOX 1 - Promoter aus Pichia a pastoris, der besonders geeignet zur induzierbaren Expression in methylotrophen Hefen geeignet ist. Vor der Induzierung der Expressionskontrollsequenz erfolgt günstigerweise eine Veränderung der Kultivierungsbedingungen derart, z.B. durch Änderung des pH-Werts auf einem Bereich von 3-4, so dass das durch die Expression der rekombinanten Nukleinsäure gebildete Trypsinogen in intakter Form im Kulturmedium angereichert wird.

Die Kultivierungsbedingungen beim erfindungsgemäßen Verfahren werden so gewählt, dass eine autokatalytische Abspaltung der Propeptidsequenz weitgehend verhindert wird. Nach Isolieren des Expressionsprodukts aus dem Kulturmedium kann die Propeptidsequenz unter kontrollierten Bedingungen abgespalten werden. Diese Abspaltung kann beispielsweise durch Zugabe von rekombinantem Trypsin oder/und durch autokatalytische Spaltung erfolgen. Unter autokatalytischer Spaltung ist dabei die selbständige Aktivierung von rekombinantem Trypsinogen, gegebenenfalls beschleunigt durch die Zugabe geringer Mengen an rekombinantem Trypsin, ohne Zusatz eines Fremdproteins zu verstehen. Auf diese Weise muss kein zusätzliches Fremdprotein, das üblicherweise aus tierischen Rohstoffquellen stammt, eingesetzt und später wieder abgereinigt werden, das bei der späteren Anwendung unerwünschte Spaltungen verursachen könnte. Die autokatalytische Aktivierung erfolgt vorzugsweise bei einem pH-Wert im Bereich von 7-8. Auf diese Weise ist gewährleistet, dass das bei der Expression entstehende Trypsinogen zunächst im stark sauren Bereich hoch aufgereinigt werden kann und gezielt die Aktivierung durch Umpufferung vorzugsweise in Gegenwart von geringen Mengen, z.B. 20 mM, $CaCl_2$ in den neutralen bis schwach basischen pH-Bereich gestartet wird. Die Aktivierung kann durch eine erneute pH-Änderung wieder in den stark sauren Bereich gestoppt werden. Die Reinigung des Trypsins kann z.B. durch Chromatographie an Ionenaustauschermaterialien wie etwa SP-Sepharose®XL oder Benzamidin-Sepharose erfolgen.

Zur Aufreinigung des Expressionsprodukts aus dem Kulturmedium kann zunächst - wie in Beispiel 6.1 beschrieben - der Kulturüberstand von den Zellen abgetrennt werden. Die nachfolgende Aufreinigung umfassend eine autokatalytische Aktivierung des Trypsinogens erfolgt vorzugsweise durch geeignete chromatographische Aufreinigungsprozeduren. In einer besonders bevorzugten Ausführungsform erfolgt - wie in Beispiel 6.2. beschrieben - eine Chromatographie ohne vorherige Abtrennung der Zellen, wobei dem die Zellen noch enthaltenden Kulturmedium ein Calciumionen enthaltender Puffer zugesetzt wird, vorzugsweise auf eine Endkonzentration von 1-30 mM Calcium. Anschließend werden die Schritte (c), (d) und gegebenenfalls (e) des erfindungsgemäßen Verfahrens, beispielsweise unter Anwendung geeigneter chromatographischer Aufreinigungsprozeduren und einem Umpufferungsschritt zur autokatalytischen Spaltung des Expressionsprodukts durchgeführt.

Eine besonders bevorzugte Ausführungsform des erfindungsgemäßen Verfahrens ist somit gekennzeichnet durch:

a) Transformation einer Wirtszelle mit einer rekombinanten Nukleinsäure mit einer für die Wirtszelle optimierten Codonnutzung, welche für die zymogene Vorstufe Trypsinogen aus Schwein kodiert, das mit einer Signalpeptidsequenz fusioniert ist und ein bis auf die Enterokinase-Erkennungssequenz verkürztes Propeptid enthält, wobei diese Spaltstelle unter bestimmten Pufferbedingungen von rekombinanten Trypsin oder autokatalytisch gespalten wird und dabei das verkürzte Trypsinogen zum aktiven Trypsin gespalten werden kann, unter der Veraussetzung, daß die rekombinante Nukleinsaure nicht mit einem naturlich vorkommenden TrypsinogenGen identisch ist,
b) Kultivierung der Wirtszelle während der Expressionsphase bei saurem pH, bevorzugt pH 3-4, so dass das verkürzte Trypsinogen löslich und sekretiert in das Kulturmedium vorliegt, jedoch die autokatalytische Aktivierung weitgehend verhindert wird,
c) Isolierung des verkürzten rekombinanten Trypsinogens aus dem Kulturüberstand und Aktivierung unter Bedingungen, die eine effektive Spaltung des verkürzten Trypsinogens durch rekombinantes Trypsin oder eine autokatalytische Spaltung erlauben und
d) gegebenenfalls Abtrennung von nicht gespaltenen Trypsinogenmolekülen von aktivem Trypsin.

Insbesondere beeinflusst die in dieser Erfindung beschriebene Verkürzung des Propeptids die Expression und die autokatalytische Aktivierung positiv. Außerdem steigt die unerwünschte weitergehende Autolyse nach erfolgter autokatalytischer Aktivierung bei einem pH >4,0 während der Wachstumsphase und der Expressionsphase der Kultur signifikant an.

Eine zusätzliche Erhöhung der Expressionsausbeute beim erfindungsgemäßen Verfahren kann dadurch erreicht werden, dass die Wirtszelle mit mehreren Vektoren, von denen jeder eine rekombinante Nukleinsäure wie zuvor angegeben enthält, transformiert wird, wobei die Vektoren unterschiedliche Selektionsmarker, z.B. Zeocin und G418 enthalten. Durch Kultivierung unter Mehrfach-Selektionsbedingungen gelingt es überraschenderweise die Expressionsausbeute von rekombinantem Trypsinogen noch deutlich zu erhöhen.

Ein weiterer Gegenstand der Beschreibung ist eine rekombinante Nukleinsäure, die für ein Trypsinogen mit einer Enterokinase-Erkennungsstelle in der Propeptidsequenz codiert, wobei die Propeptidsequenz gegenüber der natürlichen Propeptidsequenz verkürzt und vorzugsweise mit einer Signalpeptidsequenz fusioniert ist. Die erfindungsgemäß verkürzte Propeptidsequenz besteht vorzugsweise aus einer Enterokinase-Erkennungsstelle mit der Aminosäuresequenz Asp-Asp-Asp-Aps-Lys und gegebenenfalls bis zu 5 weiteren Aminosäuren N-terminal davon angeordnet. Besonders bevorzugt weistdie erfindungsgemäße Nukleinsäure die in SEQ. ID NO. 22 gezeigte Nukleotidsequenz auf.

Die Nukleinsäure ist vorzugsweise in operativer Verknüpfung mit einer regulierbaren Expressionskontrollsequenz, bei der es sich z.B. um eine für die Genexpression in Hefezellen geeignete Expressionskontrollsequenz wie etwa den AOX1-Promotor aus Pichia pastoris handelt.

Weiterhin wird ein rekombinante Vektor beschrieben, der mindestens eine Kopie einer rekombinanten Nukleinsäure wie zuvor angegeben enthält. Der Vektor ist vorzugsweise ein für die Genexpression in Hefezellen geeigneter Vektor. Beispiele für derartige Vektoren sind bei Sambrook et al., Molecular cloning: A Laboratory Manual, Cold Spring Harbor Laboratory Press, Cold Spring Harbor, New York beschrieben.

Neben der rekombinanten Nukleinsäure enthält der Vektor weitere für den jeweiligen Verwendungszweck geeignete genetische Elemente, insbesondere ein Selektionsmarkergen. Besonders bevorzugt werden Vektoren verwendet, die in der Zelle in multiplen Kopien vorliegen können, insbesondere Vektoren, die in multipler Form in das Genom der Wirtszelle integriert werden können. Beschrieben sind auch Kombinationen von Vektoren, die jeweils unterschiedliche Selektionsmarkergene enthalten und die nebeneinander in einer Wirtszelle propagierbar sind.

Außerdem wird eine rekombinante Zelle beschrieben, die mit einer Nukleinsäure oder einem Vektor transformiert ist. Vorzugsweise ist die rekombinante Zelle eine Hefezelle, insbesondere eine methylotrophe Hefezelle wie etwa Pichia pastoris oder Hansenula polymorpha.

Schließlich wird ein rekombinantes Trypsinogen beschrieben, welches von einer Nukleinsäure codiert ist. Das rekombinante Trypsinogen weist eine Propeptidsequenz auf, die gegenüber der natürliche Propeptidsequenz verkürzt ist und eine Enterokinase-Erkennungsstelle enthält. Besonders bevorzugt weist das rekombinante Trypsinogen die in SEQ. ID NO. 23 gezeigte Aminosäuresequenz auf.

Weiterhin soll die vorliegende Erfindung durch die nachfolgenden Figuren und Beispiele erläutert werden. Es zeigen:

**Figur 1:** eine Plasmidkarte des Expressionsplasmids pTRYP-9 mit dem kompletten rekombinanten Trypsinogen,

**Figur 2:** eine Plasmidkarte des Expressionsplasmids pTRYP-11 mit dem verkürzten rekombinanten Trypsinogen (sh-Trypsinogen) und dem Zeocin-Resistenzmarkergen (ZeoR),

**Figur 3:** eine Plasmidkarte des Expressionsplasmids pTRYP-13 mit dem verkürzten rekombinanten Trypsinogen (sh-Trypsinogen) und einem Kanamycin / G418 - Selektionsmarkergen (KanR).

**Beispiele:**

**Methoden:**

**Rekombinante DNA-Technik**

Zur Manipulation von DNA wurden Standardmethoden benutzt, wie sie bei Sambrook, J. et al. (1989) In. Molecular cloning: A Laboratory Manual. Cold Spring Harbor Laboratory Press, Cold Spring Harbor, New York beschrieben sind. Die verwendeten molekularbiologischen Reagenzien wurden nach den Angaben des Herstellers eingesetzt.

**Proteinbestimmung**

Die Proteinbestimmung von gereinigtem Trypsin wurde durch Extinktionsmessung bei 280 nm durchgeführt. Für E1 %/280 nm bei 1 cm Schichtdicke wird 13,6 verwendet.

Berechnung:

$$\text{Protein [mglml]} = (10\ \text{[mg/ml]}^{*}\ \Delta E_{Probe}\ ^{*}\text{Verdünnung}) / 13{,}6$$

**Pichia pastoris und Expressionsvektoren für Pichia pastoris**

Anweisungen zum Umgang mit Pichia pastoris und den Expressionsvektoren für Pichia pastoris wurden dem Katalog und den Bedienungsanleitungen von Invitrogen entnommen. Die Vektoren zur Expression des verkürzten rekombinanten Trypsinogens basieren auf die Vektoren pPICZαA und pPIC9K von Invitrogen.

**Beispiel 1:**

Gensynthese des kompletten rekombinanten Trypsinogens mit optimierter Codonverwendung für Expression in Hefe

Eine der bevorzugten Maßnahmen zur Bereitstellung des erfindungsgemäßen Verfahrens ist die Synthese einer codon-optimierten Gensequenz. Um jedes Codon zur Expression in Hefe zu optimieren war eine komplette de novo Synthese des ca. 700 Bp langen Gens, das für das komplette rekombinante Trypsinogen kodiert, erforderlich. Durch Rückübersetzung der Aminosäuresequenz des Schweine-Trypsinogens gemäß SEQ ID NO. 1 konnte durch Ausnutzung des degenerierten Codes jedes Codon, wenn erforderlich, optimiert werden. Dazu wurde das Gen in 18 Oligonukleotide mit einer Länge von 54 bis 90 Nukleotide unterteilt. Die Oligonukleotide wurden als alternierende Folge von Sinnstrang- und Gegenstrangfragmenten entworfen, die jeweils mit ihren 5'- bzw. 3'- Enden mit den benachbarten Oligonukleotiden komplementär überlappen. Der Überlappungsbereich wurde jeweils so gewählt, dass bei der Annealingreaktion in der späteren PCR-Reaktion eine unspezifische Bindung weitgehend verhindert wird. Die Oligonukleotide am 5'- bzw. 3'- Ende des Gens wurden stromaufwärts bzw, stromabwärts des kodierenden Bereichs mit Erkennungsstellen für Restriktionsendonukleasen versehen, die für eine spätere Insertion des synthetischen Gens gemäß SEQ ID NO. 2 in Expressionsvektoren genutzt werden konnten. So wurde stromaufwärts eine Erkennungsstelle für die Restriktionsendonuklease EcoRI und stromabwärts eine Erkennungsstelle für die Restriktionsendonuklease Xbal eingebaut. Die Sequenzen der Oligonukleotide sind in SEQ ID NO. 3 bis 20 dargestellt.

Die Gensynthese wurde mittels PCR-Reaktion durchgeführt. Dazu wurde der kodierende Bereich zunächst in drei Teilstücke unterteilt (Oligonukleotide 3 bis 8, 9 bis 14, 15 bis 20) und diese Teilstücke in getrennten PCR-Reaktionen mit überlappenden komplementären Oligonukleotiden erzeugt. Dabei erfolgte ein schrittweises Verlängern des Genfragmentes bis zum Volllängen-Produkt, das dann in weiteren Zyklen amplifiziert wurde. Die Annealingtemperatur wurde dabei nach dem Überlappungsbereich mit der geringsten Schmelztemperatur gewählt.

Die drei Teilstücke wurden anschließend mittels Agarosegelelektrophorese analysiert, die Produkte mit der erwarteten Länge aus dem Gel mittels QIAquick Gel Extraction Kit (Qiagen) isoliert und in einer weiteren PCR-Reaktion zu dem kompletten Genprodukt synthetisiert. Die PCR-Reaktion wurde dabei in den ersten 5 Zyklen ohne Zugabe der Primer am 5'- Ende und am 3'- Ende des gesamten Gens durchgeführt, so dass zunächst aus den drei Teilstücken wenige Fragmente des Genproduktes der erwarteten Länge entstehen. Die Annealingtemperatur wurde dabei nach dem Überlappungsbereich mit der geringsten Schmelztemperatur gewählt. Danach wurden die endständigen Primer zugegeben und die Annealingtemperatur entsprechend der Annealingtemperatur des Primers mit der geringsten Schmelztemperatur erhöht. In weiteren 25 Zyklen wurde danach das Genfragment der erwarteten Länge hochamplifiziert. Das PCR-Fragment wurde durch Sequenzierzung überprüft.

**Beispiel 2**

**Generierung des verkürzten Trypsinogen-Gens**

Durch einen speziell entworfenen 5'-Primer gemäß SEQ ID NR. 21 Wurde eine Deletion der Codons für die ersten 20 Aminosäuren des natürlicherweise vorkommenden Trypsinogens erzeugt, so dass als Sequenz des Propeptids nur noch die Codons für die Erkennungssequenz der Enteropeptidase Enterokinase Asp-Asp-Asp-Asp-Lys sowie für die notwendige Klonierung in die Expressionsvektoren für Pichia pastoris die Codons Glu-Phe, hervorgerufen durch die EcoRI-Restriktionsendonuklease-Erkennungssequenz, am N-Terminus des verkürzten rekombinanten Trypsinogens zu finden sind. Die Deletion wurde durch eine PCR-Reaktion an dem PCR-Fragment des Gens für das komplette rekombinante Trypsinogen mit dem 5'-Primer gemäß SEQ ID NR. 21 und dem 3'-Primer gemäß SEQ ID NR. 20 eingeführt. Die DNA-Sequenz bzw. die Aminosäuresequenz des verkürzten rekombinanten Trypsinogens sind in SEQ ID NR. 22 bzw. SEQ ID NR. 23 dargestellt.

**Beispiel 3**

**Klonierung der PCR-Fragmente des durch Gensynthese erzeugten kompletten rekombinanten Trypsinogens und des verkürzten rekombinanten Trypsinogens in Expressionsvektoren für Pichia pastoris**

Die PCR-Fragmente wurden mit EcoRI und Xbal (Roche Diagnostics GmbH) nachgeschnitten, nochmals isoliert (QIAquick Gel Extraction Kit/Qiagen) und anschließend in ein mit EcoRI und Xbal (Roche Diagnostics GmbH) linearisiertes und isoliertes (QIAquick Gel Extraction Kit/Qiagen) Fragment des Expressionsvektors pPICZαA (Invitrogen) ligiert. Dazu wurden jeweils 1 µl (20 ng) Vektorfragment und 3 µl (100 ng) PCR-Fragment, 1 µl 10x Ligase-Puffer (Sambrook et al., 1989 B.27), 1 µl T4 DNA-Ligase, 4 µl steriles $H_2O_{bidest.}$ pipettiert, vorsichtig gemischt und über Nacht bei 16 °C inkubiert. In diesem Vektor steht das synthetische Gen unter Kontrolle des mit Methanol (Mallinckrodt Baker B.V.) induzierbaren AOX 1-Promotors (Promotor für die Alkoholoxidase 1 aus Pichia pastoris und befindet sich im korrekten Leserahmen hinter dem Signalpeptid des α-Faktors aus Saccharomyces cerevisiae. 5 µl des Ligationsansatzes wurden dann zur Überprüfung und Plasmidgewinnung in 200 µl kompetente Zellen (Hanahan (1983) von E.coli XL1Blue (Stratagene) transformiert. Nach 30 min. Inkubation auf Eis erfolgte ein Hitzeschock (90 sec bei 42 °C). Anschließend wurden die Zellen in 1 ml LB-Medium überführt und zur phänotypischen Expression 1 Stunde bei 37 °C in LB-Medium inkubiert.

Aliquote dieses Transformationsansatzes wurden auf LB-Platten mit 100µg/ml Zeocin als Selektionsmarker ausplattiert und 15 Stunden bei 37 °C inkubiert. Von gewachsenen Klonen wurden die Plasmide isoliert (Sambrook, J. et al. (1989) In. Molecular cloning: A Laboratory Manual. Cold Spring Harbor Laboratory Press, Cold Spring Harbor, New York) und dann mittels Restriktionsanalyse und Sequenzierung auf fehlerfreie Basensequenz untersucht. Die so entstandenen Expressionsvektoren, die ein synthetisches Gen, das für die komplettes rekombinantes Trypsinogen bzw. ein verkürztes rekombinantes Trypsinogen enthält wurden pTRYP-9 (s. Fig. 1) bzw. pTRYP-11 (s. Fig. 2) genannt.

Transformation von pTRYP-9 bzw. pTRYP-11 in Pichia pastoris

Zur Transformation von pTRYP-9 bzw. pTRYP-11 in Pichia pastoris X-33, GS115 oder KM71H mit anschließender Integration in das Genom wurde der Vektor zunächst mit SacI (Roche Diagnostics GmbH) linearisiert. Die Transformation wurde mittels Elektroporation mit dem Gene Pulser II (Biorad) durchgeführt. Dazu wurde eine Kolonie von Pichia pastoris mit 5 ml YPD-Medium (Invitrogen) angeimpft und bei 30 °C über Nacht unter Schütteln inkubiert. Die Übernachtkultur wurde anschließend 1:2000 in 200 ml frisches YPD-Medium (Invitrogen) überimpft und über Nacht bei 30 °C unter Schütteln inkubiert, bis eine $OD_{600}$ von 1,3 bis 1,5 erreicht war. Die Zellen wurden abzentrifugiert (1500 x g / 5 Minuten) und das Pellet in 200 ml eiskaltem, sterilen Wasser (0 °C) resuspendiert. Die Zellen wurden wiederum abzentrifugiert (1500 x g/5 Minuten) und in 100 ml eiskaltem, sterilen Wasser (0 °C) resuspendiert. Die Zellen wurden wiederum abzentrifugiert, in 10 ml eiskaltem (0 °C) 1 M Sorbitol (ICN) resuspendiert. Die Zellen wurden wiederum abzentrifugiert, in 0,5 ml eiskaltem (0 °C) 1 M Sorbitol (ICN) resuspendiert. Die so gewonnenen Zellen wurden auf Eis gehalten und sofort zur Transformation eingesetzt. 80 µl der Zellen wurden mit ca. 1 µg linearisierter pTRYP-9 bzw. pTRYP-11-Vektor-DNA versetzt und der gesamte Ansatz in eine eiskalte (0 °C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25 µF durchgeführt. Nach Elektroporation wurde der Ansatz mit 1 ml 1M Sorbitol (ICN) versetzt und anschließend 100 bis 150 µl auf eine YPDS-Agarplatte (Invitrogen) mit 100µg/ml Zeocin (Invitrogen) ausplattiert. Die Platten wurden anschließend 2-4 Tage bei 30 °C inkubiert.

Die Klone wurden auf Raster-MD (= minimal Dextrose)-Platten überimpft und weiter analysiert. Gewachsene Klone wurden gepickt, in 20 µl sterilem Wasser resuspendiert, mit 17,5 U Lyticase (Roche Diagnostics GmbH) aufgeschlossen (1 Stunde, 30 °C, 10 min, -80 °C) und direkt mittels PCR auf die korrekte Integration der synthetischen Trypsinogen-Expressionskassette untersucht.

Klone, die die komplette Expressionskassette bei der Transformation in das Genom integriert haben, wurden dann in Expressionsversuchen eingesetzt.

Expression des kompletten bzw. verkürzten rekombinanten Trypsinogens

Positive Klone wurden in 3 ml BMGY-Medium (Invitrogen) angeimpft und über Nacht bei 30 °C unter Schütteln inkubiert. Anschließend wurden die OD bei 600 nm bestimmt und so in 10 ml BMMY-Medium (Invitrogen) pH 3,0 überimpft, dass eine $OD_{600}$ von 1 resultierte. Das BMMY-Medium (Invitrogen) pH 3,0 enthält Methanol (Mallinckrodt Baker B.V.), das die Expression des kompletten bzw. verkürzten rekombinanten Trypsinogens über den AOX 1-Promotor induziert.

Die Schüttelkolben wurden bei 30 °C unter Schütteln inkubiert, alle 24 Stunden Proben gezogen, die $OD_{600}$ bestimmt, ein Aliquot zur Untersuchung der Expression des kompletten bzw. verkürzten rekombinanten Trypsinogens mittels SDS-Polyacrylamidgelelektrophorese entnommen und jeweils mit 0,5 % Methanol (Mallinckrodt Baker B.V.) zur weiteren Induktion nachgefüttert. Die Expressionsversuche liefen über 72 Stunden.

Analyse der Expression des kompletten bzw. verkürzten rekombinanten Trypsinogens mittels SDS-Gelelektrophorese

Je 500 µl wurden der Expressionskultur entnommen, die $OD_{600}$ bestimmt und die Zellen abzentrifugiert. Der Kulturüberstand wurde aufbewahrt und das Zellpellet wurde in einer der $OD_{600}$ entsprechenden Menge Y-PER™ (50 bis 300µl/Pierce) zur Lyse resuspendiert und 1 Stunde bei Raumtemperatur geschüttelt. Anschließend wurde das Lysat zur Abtrennung von Zelltrümmern abzentrifugiert (15000 x g/5 Minuten) und der Überstand in frische Reaktionsgefäße überführt. 10 µl des Lysates und 10 µl des Kulturüberstandes wurden dann auf ein SDS-Polyacrylamidgel aufgetragen und die Proteine durch Anlegen eines elektrischen Feldes der Größe nach aufgetrennt.

Überraschenderweise konnte hier in den Kulturüberstanden sowohl bei den Klonen mit dem kompletten wie auch bei den Klonen mit dem verkürzten rekombinanten Trypsinogen eine schwache Bande identifiziert werden, die bei den Kontrollklonen nicht auftauchte. Unter Kontrollklone sind Pichia pastoris X33-Zellen zu verstehen, die mit Ausgangsvektor pPICZαA transformiert wurden und analog zu den Expressionsklonen für Trypsinogen angezogen und induziert wurden. Das Laufverhalten der neuen Proteinbande bei den Expressionsklonen stimmt mit dem errechneten Molekulargewicht überein und zeigt sich etwas höher als bovines Trypsin, das als Kontrollmarker mitaufgetragen wurde. Diese leichte Größenunterschied deutet auf ein intaktes, nicht aktiviertes Trypsinogen hin.

Überraschenderweise wurde das rekombinante verkürzte Trypsinogen deutlich stärker exprimiert als das rekombinante komplette Trypsinogen.

**Beispiel 4**

**Steigerung der Expressionsleistung durch Mehrfachtransformation**

Die besten Klone aus den Expressionsversuchen mit dem rekombinanten verkürzten Trypsinogen wurden wiederum für die Elektroporation wie oben beschrieben vorbereitet und wiederum mit 1 µg linearisierter pTRYP-11-Vektor-DNA transformiert und der Transformationsansatz auf YPDS-Agarplatten (Invitrogen) mit 1000 bis 2000 µg/ml Zeocin (Invitrogen) ausplattiert. Dadurch wird der Selektionsdruck derart erhöht, dass nur Klone wachsen können, die mehrere Kopien des Expressionsvektors pTRYP-11 und somit auch mehrere Kopien des jeweiligen Resistenzgens (hier Zeocin®) in das Genom integriert haben. Das Zeocin®-Resistenzprotein ist das Produkt des Bleomycingens von Streptoalloteichus hindustanus (Calmels, T. et al.,(1991); Drocourt, D. et al., (1990)), das Zeocin® in einem stöchiometrischen Konzentrationsverhältnis bindet und somit der Zelle Resistenz gegenüber Zeocin® verleiht. Je höher die Konzentration an Zeocin® in den YPDS-Agarplatten, um so mehr Resistenzprotein muss die Zelle erzeugen, um das Zeocin® quantitativ zu binden und damit ein Wachstum zu ermöglichen. Dies ist u.a. möglich, wenn multiple Kopien des Resistenzgens in das Genom integriert werden. Klone wurden wie oben beschrieben auf Raster-MD-Platten überimpft. Anschließend wurden diese Klone wiederum wie oben beschrieben auf Trypsinogen-Expression und -Sekretion überprüft.

Überraschenderweise konnten nach dieser Massnahme Klone mit deutlich erhöhter Expressionsleistung des verkürzten rekombinanten Trypsinogens sekretiert in den Kulturüberstand nach SDS-Polyacryamidgel identifiziert werden.

**Beispiel 5**

**Steigerung der Expressionsleistung durch Verwendung eines zweiten Selektionsdruckes**

Eine Steigerung der Zeocin®-Konzentration über 2000 µg/ml führte nicht zu einer verbesserten Expressionsleistung des verkürzten rekombinanten Trypsinogens. Um die Genkopienzahl des Gens gemäß SEQ ID NO. 22, das für das rekombinante verkürzte Trypsinogen kodiert und für die Expression in Hefe codon-optimiert ist, in den Expressionsklonen weiter zu erhöhen, wurde eine Integration zusätzlicher Expressionsvektoren in das Genom der aus Beispiel 3 und 4 hervorgegangenen Expressionsklone mit der höchsten Expressionsleistung über einen zweiten Selektionsdruck, bevorzugt G418 (Roche Diagnostics GmbH) durchgeführt.

Zu diesem Zweck wurde ein Teil der Expressionskassette aus pTRYP-11, bestehend aus einem Teil des AOX1-Promotor, dem Gen für das Signalpeptid des α-Faktors aus Saccharomyces cerevisiae, dem codon-optimierten Gen für das rekombinante verkürzte Trypsinogen gemäß SEQ ID NO:22, mit den Restriktionsendonukleasen SacI und XbaI aus pTRYP-11 ausgeschnitten, der Restriktionsansatz auf einem 1 %igen Agarosegel aufgetrennt und das 1693 bp große Fragment aus dem Gel isoliert (QIAquick Gel Extraction Kit/Qiagen). Der Vektor pPIC9K (Invitrogen) wurde gleichzeitig mit SacI und NotI geschnitten, der Restriktionsansatz auf einem 1 %igen Agarosegel aufgetrennt und das 8956 bp große Vektorfragment aus dem Gel isoliert (QIAquick Gel Extraction Kit/Qiagen). Der XbaI-Überhang des Fragmentes aus pTRYP-11 und der NotI-Überhang von pPIC9K wurde durch Klenow-Polymerase (Roche Diagnostics GmbH) nach Herstellerangaben zu stumpfen Enden aufgefüllt. Anschließend wurden die beiden so gewonnenen Fragmente wie oben beschrieben ligiert. Der Ligationsansatz wurde wie oben beschrieben in E.coli XL1 Blue (Stratagene) transformiert (die Selektion von plasmidhaltigen Klonen erfolgt hier über 100 µg/ml Ampicillin in den Nährplatten) und mittels Restriktionsanalyse und Sequenzierung überprüft. Der so entstandene Expressionsvektor wurde pTRYP-13 (s. Fig. 3) genannt.

Die Integration des Expressionsvektors pTRYP-13 in das Genom von Pichia pastoris wurde über G418 (Roche Diagnostics GmbH) selektioniert.

Die Klone mit der höchstens Trypsinogen-Expressionsleistung aus der Mehrfachtransformation mitpTRYP-11 (Zeocin-Resistenz) wurden wie oben beschrieben für die Elektroporation vorbereitet und mit 1 µg des mit SalI (Roche Diagnostics GmbH) linearisierten Vektorfragment von pTRYP-13 (G418-Resistenz) wie oben beschrieben transformiert. Der Transformationsansatz wurde anschließend 1 bis 3 Tage bei 4 °C in 1 M Sorbitol (ICN) aufbewahrt (zur Ausbildung der G418-Resistenz), dann 100 bis 200 µl auf YPD-Platten (Invitrogen) mit 1, 2 bzw. 4 mg/ml G418 (Roche Diagnostics GmbH) ausplattiert und 3 bis 5 Tage bei 30 °C inkubiert. Daraus resultierende Klone, bevorzugt von den YPD-Platten mit der höchsten G418-Konzentration, wurden wiederum wie beschrieben über SDS-Polyacrylamidgelelektrophorese auf eine erhöhte Expression des verkürzten rekombinanten Trypsinogens untersucht.

Überraschenderweise konnten nach dieser Massnahme nochmals Klone mit erhöhter Expressionsleistung des verkürzten rekombinanten Trypsinogens in den Kulturüberstand nach SDS-Polyacryamidgel identifiziert werden.

## Beispiel 6

### Isolierung des Trypsinogens aus dem Kulturüberstand und Aktivierung

6.1

Der Kulturüberstand wurde durch Mikrofiltration, Zentrifugation oder Filtration von den Zellen getrennt. Das Trypsinogen wurde durch Chromatographie an Phenylsepharose fast flow (Pharmacia) gereinigt. Die Chromatographie wurde im pH Bereich 2-4 durchgeführt. Durch Umpufferung auf pH 7-8 in Gegenwart von 20 mM $CaCl_2$ wurde die autokatalytische Aktivierung gestartet. Diese autokatalytische Aktivierung kann durch eine erneute pH-Änderung in den Bereich 2-4 wieder gestoppt werden. Durch Chromatographie an Benzamidin-Sepharose (z.B. SP-Sepharose®XL, ff) (Pharmacia/Beipackzettel) oder an einem Ionenaustauscher wurde das aktive Trypsin gereinigt. Das Trypsin wird bei pH 1,5-3 gelagert, um Autolyse zu vermeiden. Die spezifische Aktivität des gereinigten Trypsins liegt bei 180-200 U/mg Protein.

6.2

Die gesamte Fermentationsbrühe wurde im Verhältnis von etwa 1:2 bis 1:4 mit Ammoniumacetatpuffer (5-20 mM) enthaltend 5-30 mM Calciumchlorid, pH3,5, verdünnt und mittels einer "Expanded bed"-Chromatographie (McCornick (1993); EP 0 699 687) über einen Kationenaustauscher (z.B. Streamline®SP, XL) gereinigt. Dabei erfolgt die Chromatographie in Gegenwart der Zellen. Durch den Chromatographieschritt werden dann gleichzeitig die Zellen abgetrennt. Anschließend wird z.B. wie unter Beispiel 6.1 weiter verfahren (Umpufferung/Aktivierung usw.).

## Beispiel 7

### Aktivitätsbestimmung

Die Aktivität des Trypsins wurde mit Chromozym TRY (Pentapharm LTD) in 100 mM Tris pH 8,0, 20 mM $CaCl_2$ bei 25 °C bestimmt. Die Photometrische Messung erfolgt bei 405 nm.

### Abkürzungen:

YPD: Yeast Peptone Dextrose
YPDS: Yeast Peptone Dextrose Sorbitol
BMGY: Buffered Glyerol-complex medium
BMMY: Buffered Methanol-complex medium
SDS: Sodiumdodecylsulfat

### Literaturliste:

Bricteux-Gregoire, Schyns R., Florkin M (1966)
Biochim. Biophys. Acta **127**:pp 277

Calmels T., Parriche M., Durand H., Tiraby G. (1991),
Curr. Genet. **20**: pp.309

Charles M., Rovery M., Guidoni A., Desnuelle P. (1963)
Biochim. Biöphys. Acta **69**: pp115-129

Desnuelle P. (1959)

The Enzymes 2nd Edition **Vol 4** Editor Boyer, Acad. Press NY. pp. 119

Drocourt, D., Calmels T., Reynes J.P., Baron M., Tiraby G. (1990),
Nucleic Acid Research **18**: pp. 4009

Graf L., Craik C.S., Patthy A., Roczniak S., Fletterick RJ., Rutter W.J. (1987)
Biochem. **26**: pp. 2616

Graf L., Jancso A., Szilagyi L., Hegyi G., Pinter K., Naray-Szabo G., Hepp J., Medzihradszky K., Rutter W.J. (1988)
Proc. Natl. Acad. Sci USA 85, pp. 4961

Hanahan (1983)
J. Mol. Biol., **166**: pp. 557)

Higaki J.N., Evnin L.B., Craik C.S. (1989)
Biochem. **28**: pp. 9256

Hedstrom L., Szilagyi L., Rutter W.J. (1992)
Science **255:** pp.1249

Jurasek L., Fackre D. Smillie L.B. (1969)
Biochem Biophys Res Commun. **37:** pp. 99

Keil B. (1971)
The Enzymes Vol II, 3d Edition, Editor Boyer, Acad. Press NY. pp. 249-275

Light A., Savithari H.S., Liepnieks J.J. (1980)
Analytical Biochemistry **106:** pp. 199-206

McCornick, D.K., Bio/Technol. 11 (1993), 1059; Expanded Bed Absorption, Principles an Methods, Amersham Pharmacia Biotech, Edition AB, ISBN 91-630-5519-8;

Morihara K. and Tsuzuki H. (1968)
Arch Biochem Biophys **126:** pp971

Northrop J. H., Kunitz M., Herriott R. (1948)
Crystalline Enzymes, 2nd Edition, Columbia Univ. Press NY

Ryan C.A. (1965)
Arch Biochem Biophys **110:** pp169

Sambrook, J., Fritsch E.F., Maniatis T. (1989)
In. Molecular cloning: A laboratory manual. Cold Spring Harbor Laboratory Press, Cold Spring Harbor, New York

Travis J. (1968)
Biochem Biophys Res Commun. 30: pp. 730

Travis J. and Roberts R.C. (1969)
Biochemistry **8:** pp2884

Trop M. and Birk Y. (1968)
Biochem. J. **109:** pp 475

Wählby S. and Engström L. (1968)
Biochim. Biophys. Acta **151**:pp 402

Vasquez, J.R.Evnin L.B., Higaki J.N. Craik C.S. (1989)
J.Cell.Biochem. **39:** pp. 265

Wählby S. (1968)
Biochim. Biophys. Acta **151**:pp 394

Willett, W.S., Gillmor S.A., Perona J.J., Fletterick R.J., Craik C.S. (1995)
Biochem. **34:** pp.2172

Yee, L. and Blanch, H.W., (1993)
Biotechnol. Bioeng. **41:** pp. 781-790.

WO 97/00316 (NOVO NORDISK AS/Wöldike Helle, Kjeldsen Thomas)
A PROCESS FOR PRODUCING TRYPSIN

WO 99/10503 (ROCHE DIAGNOSTICS/ Kopetzki Ehrhard, Hopfner Karl-Peter, Bode Wolfram, Huber Robert)
ZYMOGENIC PROTEASE PRECURSOR THAT CAN BE AUTOCATALYTICALLY ACTIVATED AND THEIR USE.

WO 00/17332 (ELI LILLY AND COMPANY/Hanquier Jose Michael, Hershberger Charles Lee, Desplancq Dominique, Larson Jeffrey)
PRODUCTION OF SOLUBLE RECOMBINANT TRYPSINOGEN ANALOGS

WO 01 /55429 (POLYMUN SCIENTIFIC IMMUNBIOLOGISCHE FORSCHUNG GMBH/Mattanovich Diethard, Katinger Hermann, Hohenblum Hubertus, Naschberger Stefan, Weik Robert)
METHOD FOR THE MANUFACTURE OF RECOMBINANT TRYPSIN

EP 0 597 681 (ELI LILLY AND COMPANY/Greaney Michael Gerard, Rosteck Paul Robert)
EXPRESSION VECTORS FOR THE BOVINE TRYPSIN AND TRYPSINOGEN AND HOST CELLS TRANSFORMED THEREWITH

EP 0 699 687 (MITSUBISHI PHARMA CORPORATION/Noda Munehiro, Sumi Akinori, Ohmura Takao, Yokoyama Kazumasa)
PROCESS FOR PURIFYING RECOMBINANT HUMAN SERUM ALBUMIN

## Patentansprüche

1. Verfahren zur rekombinanten Herstellung von Trypsin, umfassend die Schritte:

   (a) Transformieren einer Wirtszelle mit einer rekombinanten Nukleinsäure mit einer für die Wirtszelle optimierten Codonnutzung, die für ein Trypsinogen mit einer Enterokinase-Erkennungsstelle in der Propeptidsequenz in einer von der Wirtszelle sekretierbaren Form codiert, unter der Voraussetzung, daß die rekombinante Nukleinsäure nicht mit einem naturlich vorkommenden Trypsinogen-Gen identisch ist,
   (b) Kultivieren der Wirtszelle unter Bedingungen, bei denen eine Expression der rekombinanten Nukleinsäure unter sauren Bedingungen und eine Sekretion des Expressionsprodukts in das Kulturmedium erfolgt, wobei die Bedingungen so gewählt werden, dass eine autokatalytische Abspaltung der Propeptidsequenz weitgehend verhindert wird,
   (c) Isolieren des Expressionsprodukts aus dem Kulturmedium,
   (d) Abspalten der Propeptidsequenz, wobei aktives Trypsin gebildet wird und
   (e) gegebenenfalls Abtrennen nicht gespaltener Trypsinogenmoleküle von aktivem Trypsin.

2. Verfahren nach Anspruch 1,
   **dadurch gekennzeichnet,**
   **dass** man eine Hefezelle als Wirtszelle verwendet.

3. Verfahren nach Anspruch 1 oder 2,
   **dadurch gekennzeichnet,**
   **dass** man Pichia pastoris oder Hansenula polymorpha als Wirtszelle verwendet.

4. Verfahren nach einem der Ansprüche 1 bis 3,
   **dadurch gekennzeichnet,**
   **dass** man eine rekombinante Nukleinsäure verwendet, die für ein Trypsinogen fusioniert mit einem die Sekretion vermittelnden Signalpeptid codiert.

**5.** Verfahren nach einem der Ansprüche 1 bis 4,
**dadurch gekennzeichnet,**
**dass** man eine rekombinante Nukleinsäure verwendet, die für ein Trypsinogen mit verkürzter Propeptidsequenz codiert.

**6.** Verfahren nach einem der Ansprüche 1 bis 5,
**dadurch gekennzeichnet,**
**dass** man eine rekombinante Nukleinsäure verwendet, die für ein Schweine-Trypsinogen codiert.

**7.** Verfahren nach einem der Ansprüche 1 bis 6,
**dadurch gekennzeichnet,**
**dass** man eine rekombinante Nukleinsäure mit der in SEQ. ID NO: 22 gezeigten Nukleotidsequenz verwendet.

**8.** Verfahren nach einem der Ansprüche 1 bis 7,
**dadurch gekennzeichnet,**
**dass** man die Expression der rekombinanten Nukleinsäure bei pH 3 - 4 durchführt.

**9.** Verfahren nach Anspruch 8,
**dadurch gekennzeichnet,**
**dass** das Kultivieren der Wirtszelle vor Expression der rekombinanten Nukleinsäure unter Bedingungen erfolgt, die für das Wachstum der Wirtszelle im wesentlichen optimal sind.

**10.** Verfahren nach Anspruch 9,
**dadurch gekennzeichnet,**
**dass** das Kultivieren der Wirtszelle vor Expression der rekombinanten Nukleinsäure bei pH 5 - 7 bis zu einer vorbestimmten optischen Dichte erfolgt und dann die Expression der rekombinanten Nukleinsäure induziert wird.

**11.** Verfahren nach einem der Ansprüche 1 bis 10,
**dadurch gekennzeichnet,**
**dass** man das Abspalten der Propeptidsequenz durch Zugabe von rekombinantem Trypsin oder/und durch autokatalytische Spaltung durchführt.

**12.** Verfahren nach einem der Ansprüche 1 bis 11,
**dadurch gekennzeichnet,**
**dass** man das die Wirtszellen enthaltende Kulturmedium mit einem Calciumionen enthaltenden Puffer versetzt und anschließend die Schritte (c), (d) und gegebenenfalls (e) durchführt.

## Claims

**1.** Method for the recombinant production of trypsin comprising the steps:

a) transforming a host cell with a recombinant nucleic acid having a codon usage that is optimized for the host cell which codes for a trypsinogen with an enterokinase recognition site in the propeptide sequence in a form that can be secreted by the host cell, provided that the recombinant nucleic acid is not identical to a naturally occurring trypsinogen gene,
b) culturing the host cell under conditions which enable an expression of the recombinant nucleic acid under acidic conditions and a secretion of the expression product into the culture medium, the conditions being selected such that an autocatalytic cleavage of the propeptide sequence is at least substantially prevented,
c) isolating the expression product from the culture medium,
d) cleaving the propeptide sequence to form active trypsin and
e) optionally separating non-cleaved trypsinogen molecules from active trypsin.

**2.** Method as claimed in claim 1,
**characterized in that**,
a yeast cell is used as the host cell.

**3.** Method as claimed in claim 1 or 2,

**characterized in that**
Pichia pastoris or Hansenula polymorpha is used as the host cell.

4. Method as claimed in one of the claims 1 to 3,
**characterized in that**
a recombinant nucleic acid is used which codes for a trypsinogen fused to a signal peptide that mediates secretion.

5. Method as claimed in one of the claims 1 to 4,
**characterized in that**
a recombinant nucleic acid is used which codes for a trypsinogen having a shortened propeptide sequence.

6. Method as claimed in one of the claims 1 to 5,
**characterized in that**
a recombinant nucleic acid is used which codes for a porcine trypsinogen.

7. Method as claimed in one of the claims 1 to 6,
**characterized in that**
a recombinant nucleic acid is used having the nucleotide sequence shown in SEQ ID NO. 22.

8. Method as claimed in one of the claims 1 to 7,
**characterized in that**
the recombinant nucleic acid is expressed at pH 3 - 4.

9. Method as claimed in claim 8,
**characterized in that**
the host cell is cultured before expression of the recombinant nucleic acid under conditions that are essentially optimal for the growth of the host cell.

10. Method as claimed in claim 9,
**characterized in that**
the host cell is cultured before expression of the recombinant nucleic acid at pH 5 - 7 up to a predetermined optical density and then the expression of the recombinant nucleic acid is induced.

11. Method as claimed in one of the claims 1 to 10,
**characterized in that**
the propeptide sequence is cleaved by addition of recombinant trypsin or/and by autocatalytic cleavage.

12. Method as claimed in one of the claims 1 to 11,
**characterized in that**
the culture medium containing the host cells is admixed with a buffer containing calcium ions and subsequently the steps (c), (d) and optionally (e) are carried out.

## Revendications

1. Procédé de production de trypsine par recombinaison, comprenant les étapes :

(a) transformation d'une cellule hôte avec un acide nucléique recombiné avec un usage des codons optimisé pour la cellule hôte, qui code un trypsinogène ayant un site de reconnaissance pour une entérokinase dans la séquence propeptidique sous une forme pouvant être sécrétée par la cellule hôte, avec la condition que l'acide nucléique recombiné ne soit pas identique à un gène de trypsinogène naturel,
(b) culture de la cellule hôte dans des conditions dans lesquelles il se produit une expression de l'acide nucléique recombiné dans des conditions acides et une sécrétion du produit d'expression dans le milieu de culture, où les conditions sont choisies de telle manière qu'un clivage autocatalytique de la séquence propeptidique est largement évité,
(c) isolement du produit d'expression à partir du milieu de culture,
(d) clivage de la séquence propeptidique, où de la trypsine active est formée et
(e) éventuellement séparation des molécules de trypsinogène non clivées de la trypsine active.

2. Procédé selon la revendication 1 **caractérisé en ce que** l'on utilise une cellule de levure comme cellule hôte.

3. Procédé selon la revendication 1 ou 2 **caractérisé en ce que** l'on utilise Pichia pastoris ou Hansenula polymorpha comme cellule hôte.

4. Procédé selon l'une des revendications 1 à 3 **caractérisé en ce que** l'on utilise un acide nucléique recombiné qui code un trypsinogène fusionné à un peptide signal permettant la sécrétion.

5. Procédé selon l'une des revendications 1 à 4 **caractérisé en ce que** l'on utilise un acide nucléique recombiné qui code un trypsinogène à séquence propeptidique raccourcie.

6. Procédé selon l'une des revendications 1 à 5 **caractérisé en ce que** l'on utilise un acide nucléique recombiné qui code un trypsinogène de porc.

7. Procédé selon l'une des revendications 1 à 6 **caractérisé en ce que** l'on utilise un acide nucléique recombiné ayant la séquence nucléotidique montrée dans SEQ. ID NO : 22.

8. Procédé selon l'une des revendications 1 à 7 **caractérisé en ce que** l'on réalise l'expression de l'acide nucléique recombiné à pH 3-4.

9. Procédé selon la revendication 8 **caractérisé en ce que** la culture de la cellule hôte avant l'expression de l'acide nucléique recombiné a lieu dans des conditions qui sont sensiblement optimales pour la croissance de la cellule hôte.

10. Procédé selon la revendication 9 **caractérisé en ce que** la culture de la cellule hôte avant l'expression de l'acide nucléique recombiné a lieu à pH 5-7 jusqu'à ce qu'une densité optique prédéterminée apparaisse après quoi l'expression de l'acide nucléique recombiné est induite.

11. Procédé selon l'une des revendications 1 à 10 **caractérisé en ce que** l'on réalise le clivage de la séquence propeptidique par addition de trypsine recombinée et/ou par clivage autocatalytique.

12. Procédé selon l'une des revendications 1 à 11 **caractérisé en ce que** l'on ajoute au milieu de culture contenant les cellules hôtes un tampon contenant des ions calcium après quoi on accomplit les étapes (c), (d) et éventuellement (e).

Fig. 1

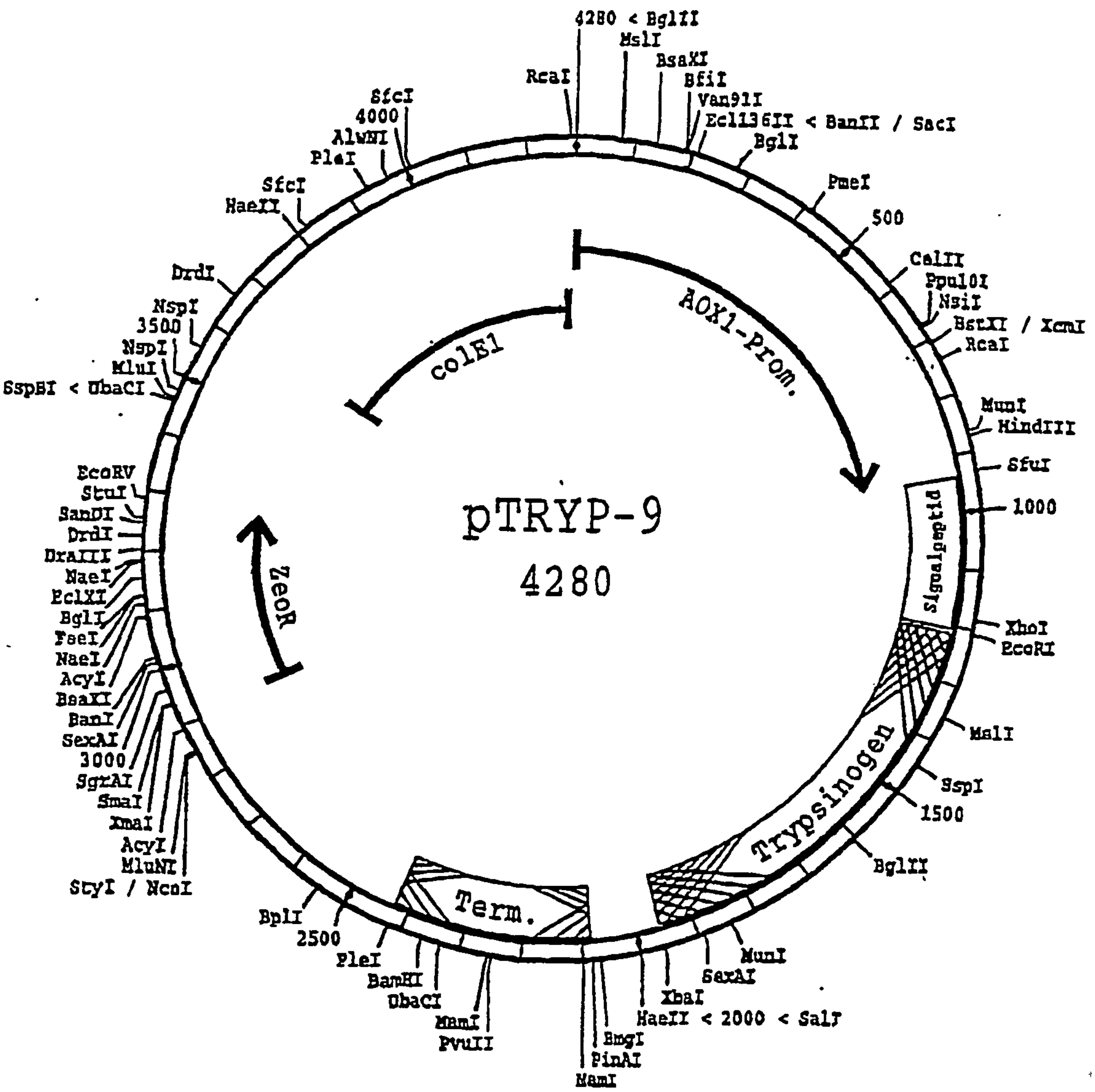
pTRYP-9
4280
4280 < BglII
MslI
BsaXI
BfiI
Van91I
Ecl136II < BanII / SacI
BglI
PmeI
500
CelII
Ppu10I
NsiI
BstXI / XcmI
RcaI
MunI
HindIII
SfuI
1000
XhoI
EcoRI
MslI
SspI
1500
BglII
MunI
SexAI
XbaI
HaeII < 2000 < SalI
BmgI
PinAI
MamI
PvuII
MamI
UbaCI
BamHI
PleI
2500
BplI
StyI / NcoI
MluNI
AcyI
XmaI
SmaI
SgrAI
3000
SexAI
BanI
BsaXI
AcyI
NaeI
FseI
BglI
EclXI
NaeI
DraIII
DrdI
SanDI
StuI
EcoRV
SspBI < UbaCI
MluI
NspI
3500
NspI
DrdI
HaeII
SfcI
PleI
AlwNI
4000
SfcI
RcaI
AOX1-Prom.
Signalpeptid
Trypsinogen
Term.
ZeoR
colE1

Figur 2

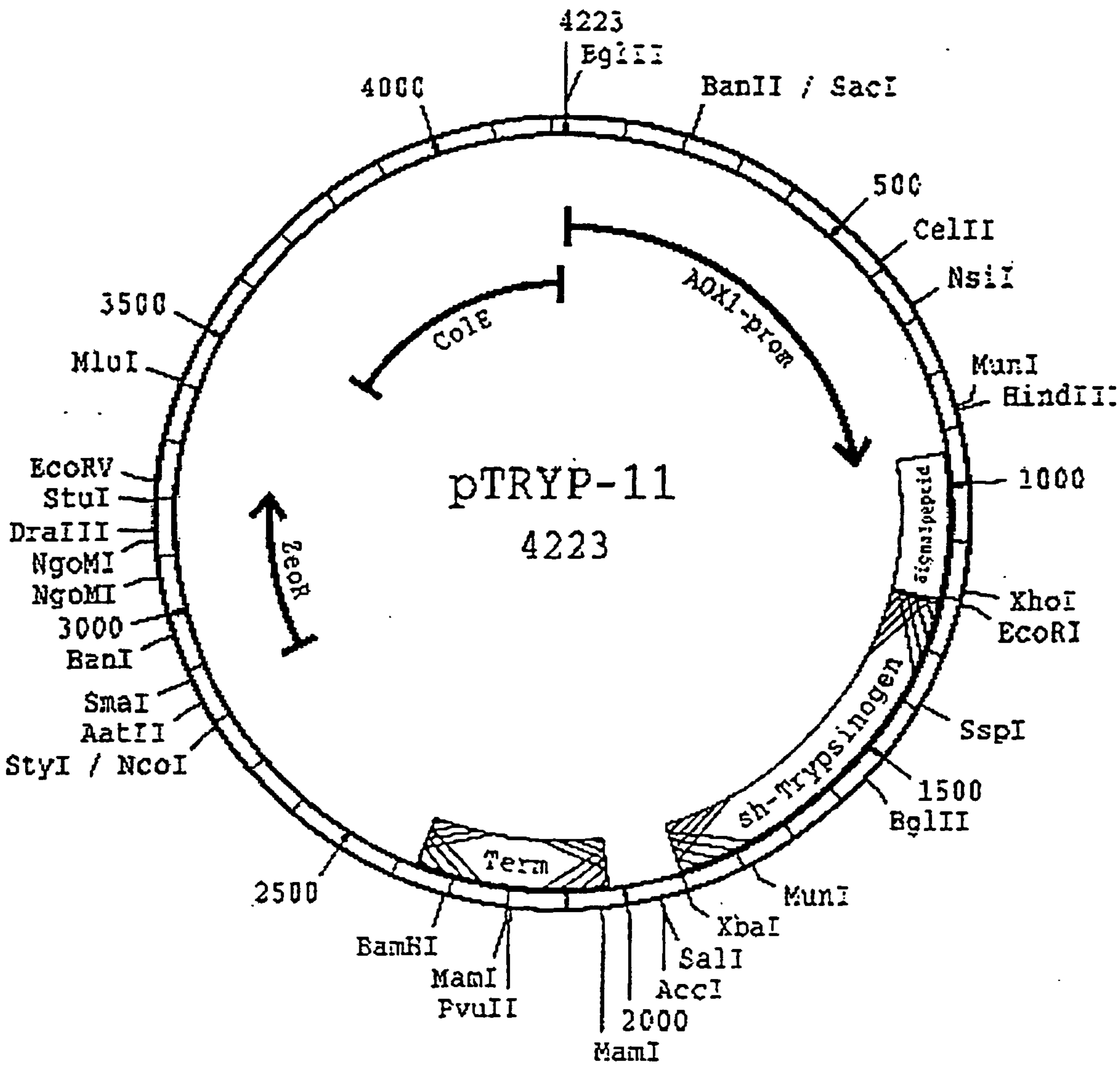
4223
BglII
BanII / SacI
500
CelII
NsiI
MunI
HindIII
1000
XhoI
EcoRI
SspI
1500
BglII
MunI
XbaI
SalI
AccI
2000
MamI
BamHI
MamI
PvuII
2500
StyI / NcoI
AatII
SmaI
BanI
3000
NgoMI
NgoMI
DraIII
StuI
EcoRV
MluI
3500
4000
AOX1-prom
signalpeptid
sh-Trypsinogen
Term
ZeoR
ColE
pTRYP-11
4223

Figur 3

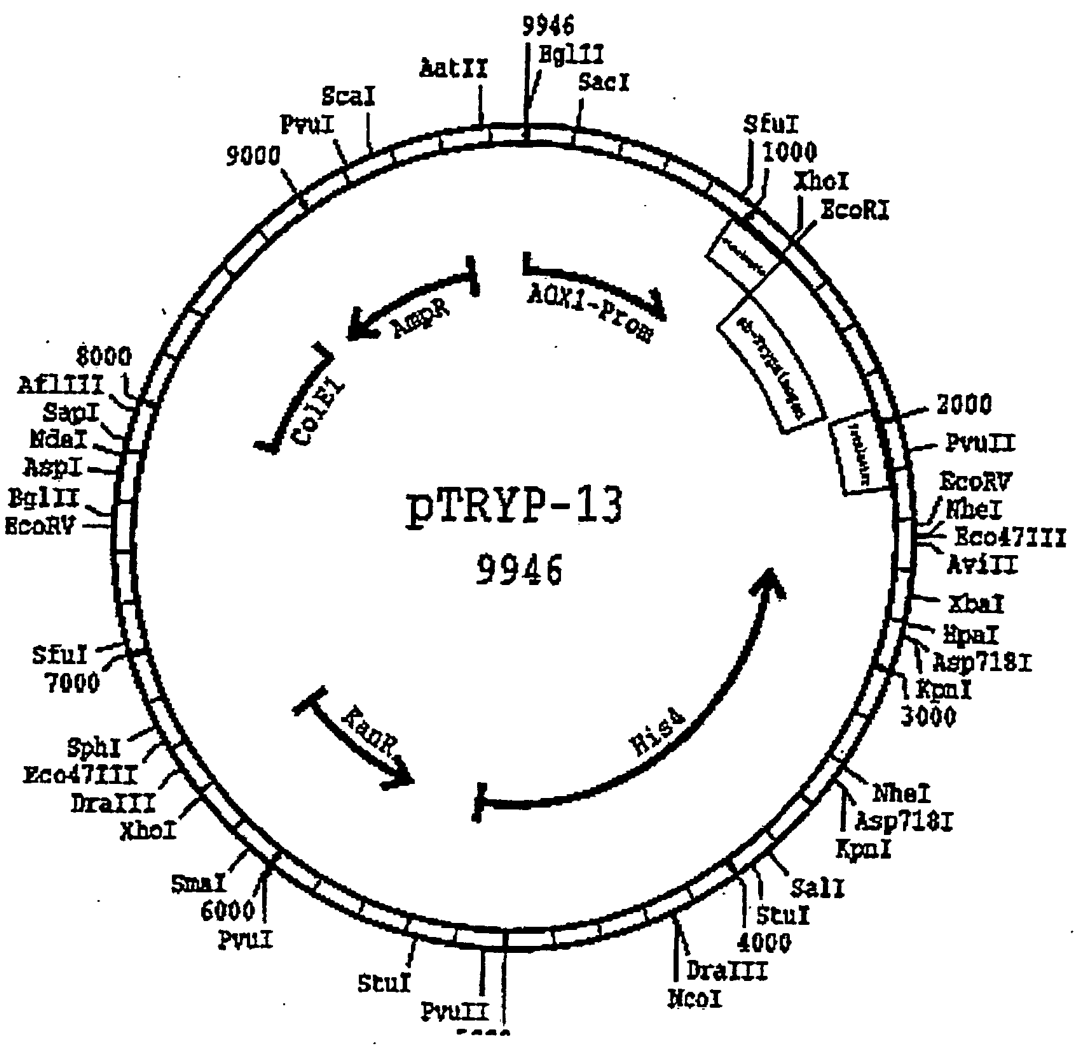
9946
BglII
SacI
AatII
ScaI
PvuI
9000
SfuI
1000
XhoI
EcoRI
AOX1-Prom
AmpR
ColE1
8000
AflIII
SapI
NdaI
AspI
BglII
EcoRV
2000
PvuII
EcoRV
NheI
Eco47III
AvrII
XbaI
HpaI
Asp718I
KpnI
3000
pTRYP-13
9946
SfuI
7000
KanR
His4
SphI
Eco47III
DraIII
XhoI
NheI
Asp718I
KpnI
SalI
StuI
4000
DraIII
NcoI
SmaI
6000
PvuI
StuI
PvuII